# FASCICULE DE BREVET EUROPEEN

(11) **EP 4 216 821 B1**
(45) Date de publication et mention de la délivrance du brevet: **13.08.2025**
(21) Numéro de dépôt: 21801139.3
(22) Date de dépôt: 03.11.2021
(51) Int. Cl.: A61N 1/04, A63B 21/002, A61N 1/36, A61B 5/00, A61B 5/22, A61B 5/11

(54) **DISPOSITIF ET PROCEDE DE MESURE D'UNE FORCE MUSCULAIRE D'UN PATIENT**
VORRICHTUNG UND VERFAHREN ZUR MESSUNG DER MUSKELKRAFT EINES PATIENTEN
DEVICE AND METHOD FOR MEASURING A PATIENT'S MUSCLE STRENGTH

(30) Priorité: 06.11.2020 BE 202005792; 06.11.2020 US 202017091468; 09.06.2021 US 202117342903; 09.06.2021 US 202117342924
(43) Date de publication de la demande: 02.08.2023
(73) Titulaire: Myocene, 4020 Liège (BE)
(72) Inventeur: RIGAUX, Pierre, 4020 Liège (BE); MIGNOLET, Jean-Yves, 4350 Momalle (BE)
(74) Mandataire: Gevers Patents
(86) Numéro de dépôt international: PCT/EP2021/080477
(87) Numéro de publication internationale: WO 2022/096489

(56) Documents cités:
- US-B1- 9 114 255
- VERKERKE G J ET AL: "Precision, comfort and mechanical performance of the Quadriso-tester, a quadriceps force measuring device", MEDICAL & BIOLOGICAL ENGINEERING & COMPUTING, SPRINGER, BERLIN, DE, vol. 41, no. 3, 1 May 2003 (2003-05-01), pages 283 - 289, XP019834565, ISSN: 1741-0444
- DOUMA K. W. ET AL: "Reliability of the Q Force; a mobile instrument for measuring isometric quadriceps muscle strength", BMC SPORTS SCIENCE, MEDICINE AND REHABILITATION, vol. 8, no. 1, 19 February 2016 (2016-02-19), XP055876339, Retrieved from the Internet <URL:https://link.springer.com/content/pdf/10.1186/s13102-016-0029-x.pdf> [retrieved on 20220105], DOI: 10.1186/s13102-016-0029-x
- MARTIN V. ET AL: "Assessment of low-frequency fatigue with two methods of electrical stimulation", JOURNAL OF APPLIED PHYSIOLOGY, vol. 97, no. 5, 1 November 2004 (2004-11-01), US, pages 1923 - 1929, XP055814641, ISSN: 8750-7587, DOI: 10.1152/japplphysiol.00376.2004

## Description

### Domaine technique

La présente invention concerne un dispositif et un procédé de mesure d'une force musculaire d'un patient.

### Art antérieur

Pour réaliser des mesures de force d'un muscle, tel que le quadriceps, plusieurs techniques sont généralement utilisées.

Une première technique utilisée est un appareil isocinétique, dans lequel le patient est installé sur un siège et sanglé à celui-ci. Le membre inférieur est attachée à un bras articulé relié à un moteur et un dynamomètre qui sont reliés à la chaise. Le mouvement du patient est entravé de façon à ce qu'il se déroule à vitesse constante. Une seconde technique qualifiée d'isotonique consiste en l'utilisation de charges lourdes que le sujet doit déplacer. Semblablement à un banc de musculation, le patient est installé sur un siège ou un banc muni d'un bras articulé, relié soit directement soit par l'intermédiaire de poulies, à des charges (poids, plaques métalliques de fonte, etc.). L'inconvénient de ces techniques est que les appareils utilisés pour la mise en œuvre de ces techniques sont lourds et/ou encombrants, et prévus pour être installés de façon permanente dans une pièce dédiée à leur usage. Un patient désireux de faire une mesure doit donc se rendre dans un endroit particulier.

Une troisième technique permet de mesurer uniquement la force isométrique au moyen d'un dynamomètre manuel. Un opérateur distinct du patient tient le dynamomètre dans sa main grâce à une sangle et exerce une force contraire à celle du patient. Dans certains cas le dynamomètre est fixé via des sangles au pied d'une table. Cette technique est peu pratique d'utilisation et les mesures obtenues sont peu précises. Lorsque l'opérateur tient le dynamomètre, l'aspect isométrique n'est pas garanti, car il est dépendant du mouvement du patient et de la pression exercée par l'opérateur. La mesure est approximative. L'opérateur doit en outre être capable d'exercer une force supérieure au patient, ce qui est rarement le cas lorsqu'on mesure la force isométrique maximale du quadriceps d'un sportif confirmé. Cette technique est donc plutôt limitée à la rééducation. Lorsque le dynamomètre est fixé à une table, la mise en place au moyen de sangles est généralement difficile, ce qui engendre aussi une mesure très imprécise et peu reproductible.

**Le** document JP 2019-180555 A divulgue un dispositif de mesure de la force musculaire des membres inférieurs. Ce dispositif comprend un cadre, un bras avec une extrémité fixée au cadre et une partie d'assise montée sur le cadre, la partie d'assise comprenant une surface d'assise et un dossier. **Un** coussin est attaché au cadre devant la partie d'assise de sorte à supporter l'arrière d'un genou d'un sujet assis sur la partie d'assise ; une partie coussin « jambe » est fixée au bras à une position correspondant à une partie de la jambe sous le genou du patient lorsqu'il est assis sur la partie d'assise. Le dispositif comprend également un instrument pour mesurer au moins une force musculaire exercée en poussant vers le bas (ou vers le haut) la partie coussin « jambe » avec la surface arrière (ou avant) de la jambe du patient lorsqu'il est assis sur la partie d'assise, la partie coussin « genou » servant de point d'appui.

L'inconvénient du dispositif de ce document est que sa structure est complexe et lourde. Ce dispositif n'est donc pas facile à transporter.

Le document US 9,114,255 B1 divulgue une orthèse destinée à rééduquer ou à exercer l'articulation du genou d'un sujet. L'orthèse comprend une plateforme étroite et allongée pour accueillir une partie supérieure d'un membre inférieur d'un sujet, couplée à un cadre s'étendant inférieurement à partir de la plateforme et maintenant un élément transverse, ayant une forme d'un rondin, prévu pour être positionné devant le tibia du sujet. L'orthèse comprend un capteur dans le cadre, au niveau de la plateforme, pour détecter une force indirecte représentative d'une force exercée par le membre inférieur durant une rééducation ou un exercice.

Cette orthèse ne permet pas d'obtenir des mesures précises de la force qui est réellement exercée par le membre inférieur, et ce notamment car elle n'induit pas une bonne stabilité durant son utilisation.

Les publications scientifiques :
- VERKERKE G J ET AL, "Precision, comfort and mechanical performance of the Quadriso-tester, a quadriceps force measuring device", MEDICAL & BIOLOGICAL ENGINEERING & COMPUTING, SPRINGER, BERLIN (DE), vol. 41, no. 3 (2003-05-01), ISSN 1741-0444, pages 283-289, XP19834565 ;
- DOUMA K. W. ET AL, "Reliability of the Q Force; a mobile instrument for measuring isometric quadriceps muscle strength", BMC SPORTS SCIENCE, MEDICINE AND REHABILITATION, vol. 8, no. 1 (2016-02-19), XP55876339 ;
divulguent des mesures expérimentales d'une force musculaire d'un humain au moyen d'un dispositif comprenant un banc ou un fauteuil, une extension inférieure d'appui d'un membre de l'humain et un instrument de mesure.

Le banc ou le fauteuil comprend un corps mécaniquement lourd et rigide afin de garantir le maintien des dispositifs pendant les mesures. En outre, le placement ou le changement de position du membre nécessite de démonter partiellement le dispositif. Ce dernier est donc peu maniable et difficile à transporter.

La publication MARTIN V. ET AL, "Assessment of low-frequency fatigue with two methods of electrical stimulation", JOURNAL OF APPLIED PHYSIOLOGY, vol. 97, no. 5, 1 November 2004 (2004-11-01), pages 1923-1929, XP55814641, expose quant à elle des observations de mesures de forces musculaires en utilisant des électrostimulations à plusieurs fréquences. Une détermination précise de la fatigue musculaire ne peut cependant pas être dérivée de ces observations.

Il y a donc un besoin pour un dispositif de mesure d'une force musculaire d'un patient qui soit plus simple et plus pratique à transporter tout en fournissant des mesures précises.

### Résumé de la divulgation

L'invention est décrite dans l'ensemble de revendications joint en annexe.

### Exposé de de la divulgation

À cet effet, la divulgation propose un dispositif de mesure d'une force musculaire d'un membre inférieur d'un patient comprenant une assise pour recevoir le patient en position assise et apte à être posée sur un support essentiellement horizontal, un élément d'appui du membre inférieur pour recevoir au moins une partie du membre inférieur du patient, couplé mécaniquement à l'assise, un instrument de mesure pour mesurer de façon directe une force exercée par le membre inférieur au niveau de l'élément d'appui du membre inférieur, le dispositif de mesure étant configuré de sorte qu'il reste essentiellement immobile par rapport au support lors de l'application d'une force par le membre inférieur du patient au niveau de l'élément d'appui du membre inférieur, grâce au poids du patient s'exerçant au niveau de l'assise.

Dans un mode de réalisation non couvert par la présente invention, l'instrument peut être fixé (directement, sans intermédiaire) sur une pièce agencée pour être alignée avec la direction de la force exercée par le membre inférieur au niveau de l'élément d'appui du membre inférieur (dans une (ou en) configuration d'utilisation du dispositif). En d'autres termes, de façon à la fois équivalente et substituable, l'instrument peut être fixé (directement, sans intermédiaire) sur une pièce alignée avec la direction de la force, dans une (ou en) configuration d'utilisation du dispositif. Typiquement, l'instrument peut être destiné à être aligné avec la direction de la force, dans une configuration d'utilisation du dispositif. De préférence, l'instrument de mesure est agencé devant ou derrière le membre inférieure, plus préférentiellement, au moins partiellement aligné avec un point d'appui de la force le long de la direction de la force, de telle sorte qu'il travaille en traction ou en compression en configuration d'utilisation du dispositif.

Ces caractéristiques du précédent paragraphe concernant le positionnement de l'instrument sont substituées selon l'invention par le fait que l'instrument de mesure soit une même pièce avec l'élément d'appui, la pièce à laquelle est fixé l'instrument n'est autre que l'élément d'appui.

De façon générale, l'instrument du dispositif est de préférence agencé pour permettre une mesure directe de la force exercée par le membre inférieur au niveau de l'élément d'appui du membre inférieur.

Le terme « aligné » utilisé ci-dessus (et dans le cadre de ce document) est typiquement à interpréter comme le fait que la pièce (et/ou l'instrument) s'étend le long (et de préférence partiellement ou totalement symétriquement autour) de la direction de la force (considérée comme vecteur physique avec son point d'appui). De préférence, une distance entre la pièce (et/ou l'instrument) et l'élément d'appui est inférieure à 10 cm.

Dans le cadre de ce document, tel qu'il sera compris d'un homme du métier, « le poids du patient » correspond généralement à tout le poids du patient. Comme l'assise permet de recevoir le patient en position assise, et que le poids du patient s'exerce au niveau de celle-ci, le dispositif est ainsi particulièrement stable lors de son utilisation, y compris lorsque la force exercée au niveau de l'élément d'appui est grande (par exemple, lors d'une contraction par électrostimulation d'un muscle quadriceps).

Optionnellement, l'assise est un plateau s'étendant continument d'au moins 40 centimètres selon deux axes perpendiculaires. De préférence, le plateau a une forme rectangulaire et a des côtés d'une longueur comprise entre 40 et 70 cm.

Optionnellement, le dispositif comprend au moins un bras couplant mécaniquement l'élément d'appui du membre inférieur à l'assise.

Optionnellement, l'angle entre le ou les bras et l'assise est réglable dans une configuration d'utilisation du dispositif.

Optionnellement, le ou les bras sont pliés contre l'assise dans une configuration de transport du dispositif.

Optionnellement, le dispositif comprend un seul élément d'appui du membre inférieur, ajustable sur le ou les bras pour s'adapter à différents membres inférieurs.

Optionnellement, le ou les bras déportent l'élément en dehors du plan de l'assise, sous l'assise, en configuration d'utilisation du dispositif.

Optionnellement, le ou les bras sont mobiles en translation latéralement par rapport au patient en position assise. De façon semblable et optionnelle, le ou les bras sont mobiles en translation latéralement par rapport à l'assise.

Optionnellement, l'instrument de mesure est une jauge de contrainte ou un dynamomètre mécanique.

Optionnellement, l'instrument de mesure est adapté à travailler en compression ou en traction.

Optionnellement, le dispositif comprend en outre des organes pour un positionnement amovible de l'assise sur le support.

Optionnellement, les organes de positionnement sont réglables en hauteur.

Optionnellement, le dispositif est configuré de sorte que la force exercée par le membre inférieur au niveau de l'élément d'appui du membre inférieur est essentiellement parallèle à l'assise, dans une configuration d'utilisation du dispositif.

Optionnellement, le dispositif est configuré de sorte que l'élément d'appui du membre inférieur est apte à recevoir une partie du membre inférieur sous le genou du patient.

Optionnellement, l'élément d'appui comprend une anse conformée pour s'adapter à la partie du membre inférieur en appui contre l'élément et/ou comprend une sangle d'immobilisation du membre inférieur contre l'élément d'appui. Optionnellement et de façon semblable, l'élément d'appui est une telle anse comprenant une partie arrondie configurée pour épouser la courbure de la partie du membre inférieur et immobiliser latéralement le membre inférieur, dans une configuration d'utilisation du dispositif.

Optionnellement, le muscle dont la force est mesurée est un quadriceps.

Optionnellement, le support est une table.

De façon semblable, l'invention propose également un dispositif de mesure d'une force musculaire d'un membre inférieur d'un patient comprenant
- une assise pour recevoir le patient en position assise et apte à être posée sur un support essentiellement horizontal,
- un élément d'appui du membre inférieur pour recevoir au moins une partie du membre inférieur du patient, couplé mécaniquement à l'assise par un bras mécanique ou un cadre mécanique,
- un instrument de mesure pour mesurer une force exercée par le membre inférieur au niveau de l'élément d'appui du membre inférieur,
le bras mécanique ou le cadre mécanique comprenant un organe de liaison à l'instrument au niveau de l'élément d'appui,
le dispositif de mesure étant configuré de sorte qu'il reste essentiellement immobile par rapport au support lors de l'application d'une force par le membre inférieur du patient au niveau de l'élément d'appui du membre inférieur, grâce au poids du patient s'exerçant au niveau de l'assise.

**Il** s'agit d'un mode de réalisation du dispositif tel que présenté de façon plus générale au premier paragraphe de l'exposé de l'invention, de sorte qu'ils partagent les mêmes avantages. Le fait l'instrument soit lié au bras mécanique ou au cadre mécanique au niveau de l'élément d'appui induit que l'instrument est forcément agencé au niveau de l'élément d'appui, et donc au plus près de l'endroit où la force est exercée, typiquement aligné avec la force, ce qui permet des mesures directes de la force, qui sont donc plus précises.

Avantageusement, la structure du dispositif est alors très simple et légère. Le bras ou le cadre peut avoir une forme simple, par exemple une forme projetée en "I", "L", "T", "U", "S" ou "Z" dans au moins un plan orthogonal à l'assise, et de préférence comprendre au moins une extrémité haute couplée (ou fixée) à l'assise, et au moins une extrémité basse couplée (ou fixée) à l'élément d'appui.

En particulier, le cas où un cadre mécanique couple l'assise avec l'élément d'appui est un cas particulier de « le ou les bras » mentionné ci-dessus.

À ces égards, les précédents modes de réalisation et options, ainsi que leurs avantages respectifs, s'étendent mutatis mutandis au dispositif comprenant un bras ou un cadre mécanique tel que décrit ci-dessus.

L'invention se rapporte aussi à un procédé de mesure d'une force musculaire d'un membre inférieur d'un patient, comprenant les étapes de fourniture du dispositif décrit précédemment, de mise en place de l'assise sur un support essentiellement horizontal, de positionnement du patient assis sur l'assise, de positionnement d'un membre inférieur du patient dont la force est à mesurer dans l'élément d'appui du membre inférieur, de prise de mesures de force du membre inférieur au niveau de l'élément d'appui du membre inférieur.

Optionnellement, le positionnement du patient est tel que son pied soit à distance du sol.

Optionnellement, l'entièreté d'une cuisse du patient repose sur l'assise et l'arrière de son genou est en contact d'un bord de l'assise.

Optionnellement, le procédé comprend en outre une étape de positionnement de l'élément d'appui du membre inférieur en regard du membre inférieur dont la force musculaire est à mesurer.

Optionnellement, le procédé comprend en outre un réglage de l'angle entre l'assise et au moins un bras couplant mécaniquement l'élément d'appui du membre inférieur à l'assise et/ou comprend le réglage en translation du au moins un bras latéralement par rapport au patient assis sur l'assise.

Optionnellement, le support est une table.

Optionnellement, le procédé est pour mesurer une fatigue musculaire d'un quadriceps ou ischio-jambier d'un patient.

Optionnellement, le procédé comprend en outre une étape d'électrostimulation engendrant une force exercée par le membre inférieur au niveau de l'élément d'appui du membre inférieur.

Optionnellement, le procédé comprend en outre les étapes d'électrostimulations d'un muscle du membre inférieur par exemple un quadriceps ou un ischio-jambier, à différentes fréquences, de prise de mesures de forces du membre inférieur au niveau de l'élément d'appui du membre inférieur en réponse aux électrostimulations susdites, de détermination d'une fatigue musculaire sur bases des mesures de forces du membre inférieur prises en réponse aux électrostimulations susdites.

Optionnellement, les différentes fréquences comprennent une première et une deuxième fréquences, différant d'au moins 10% l'une de l'autre, et les mesures de forces comprennent une première mesure de force du membre inférieur au niveau de l'élément d'appui du membre inférieur en réponse à l'électrostimulation du muscle à la première fréquence, et une deuxième mesure de force du membre inférieur au niveau de l'élément d'appui du membre inférieur en réponse à l'électrostimulation du muscle à la deuxième fréquence.

Optionnellement, la détermination de la fatigue musculaire comprend un calcul de ratio entre la première et la deuxième mesures de forces et est basée au moins sur une comparaison entre ce ratio et un seuil.

Optionnellement, la première fréquence est comprise entre 0 et 50 Hz, la deuxième fréquence est comprise entre 50 et 150 Hz, et le seuil est compris entre 50 et 100%.

Optionnellement, la première fréquence est inférieure à 50 Hz, les différentes fréquences comprennent une famille de fréquences inférieures à 200 Hz et multiples entiers de la première fréquence, et la détermination de la fatigue musculaire est basée sur un calcul d'une intégrale discrète d'une fonction associant à chaque fréquence de la famille une mesure de force prise au niveau de l'élément d'appui du membre inférieur en réponse à l'électrostimulation à cette fréquence.

L'usage, dans ce document, du verbe « comprendre », de ses variantes, ainsi que ses conjugaisons, ne peut en aucune façon exclure la présence d'éléments autres que ceux mentionnés. L'usage, dans ce document, de l'article indéfini « un », « une », ou de l'article défini « le », « la » ou « l' », pour introduire un élément n'exclut pas la présence d'une pluralité de ces éléments.

Les termes « premier », « deuxième », etc. sont, quant à eux, utilisés dans le cadre de ce document exclusivement pour différencier différents éléments, et ce sans impliquer d'ordre entre ces éléments.

L'ensemble des modes de réalisation préférés ainsi que l'ensemble des avantages du dispositif selon chaque exemple de l'invention se transposent mutatis mutandis aux autres exemples de l'invention et au présent procédé.

### Brève description des figures

D'autres caractéristiques et avantages de la présente invention apparaîtront à la lecture de la description détaillée qui suit pour la compréhension de laquelle on se reportera aux figures annexées qui montrent :
- la figure 1, une vue en perspective d'un exemple du dispositif selon une variante de l'invention ;
- la figure 2, une vue de profil du dispositif de la figure 1 ;
- la figure 3, une vue en perspective d'un exemple d'élément d'appui du dispositif et d'instrument de mesure ;
- la figure 4, une vue en perspective d'un mode de réalisation préféré du dispositif selon l'invention ;
- la figure 5, une vue en perspective d'un exemple d'instrument de mesure.

Les dessins des figures ne sont pas à l'échelle. Des éléments semblables sont en général dénotés par des références semblables dans les figures. Dans le cadre du présent document, les éléments identiques ou analogues peuvent porter les mêmes références. En outre, la présence de numéros ou lettres de référence aux dessins ne peut être considérée comme limitative, y compris lorsque ces numéros ou lettres sont indiqués dans les revendications.

### Description détaillée de modes de réalisation de l'invention

L'invention se rapporte à un dispositif de mesure d'une force musculaire d'un membre inférieur d'un patient comprenant une assise pour recevoir le patient en position assise et apte à être posée sur un support essentiellement horizontal ainsi qu'un élément d'appui du membre inférieur pour recevoir au moins une partie du membre inférieur du patient, l'élément étant couplé mécaniquement à l'assise. Le dispositif comprend également un instrument de mesure pour mesurer une force exercée par le membre inférieur au niveau de l'élément d'appui du membre inférieur. Le dispositif de mesure est configuré de sorte qu'il reste essentiellement immobile par rapport au support lors de l'application d'une force par le membre inférieur du patient au niveau de l'élément d'appui du membre inférieur, grâce au poids du patient s'exerçant au niveau de l'assise.

Le dispositif est conçu pour être utilisé sans opérateur et de structure stable et rigide pour que les mesures soient précises et reproductibles. Le dispositif est simple et léger. L'assise se dépose sur n'importe quel support tel qu'une table ou une chaise. Le dispositif est donc très facilement transportable et il permet de réaliser des mesures précises sans besoin d'équipement supplémentaire ; le dispositif étant léger, il est portatif et peut être amené facilement dans n'importe quel lieu où se trouvent des patients sur lesquels on veut faire les mesures de force musculaire. Le dispositif se déplace où se trouvent les patients qui ne doivent donc pas se rendre dans un endroit particulier. La précision des mesures permet une prise en charge adéquate des patients.

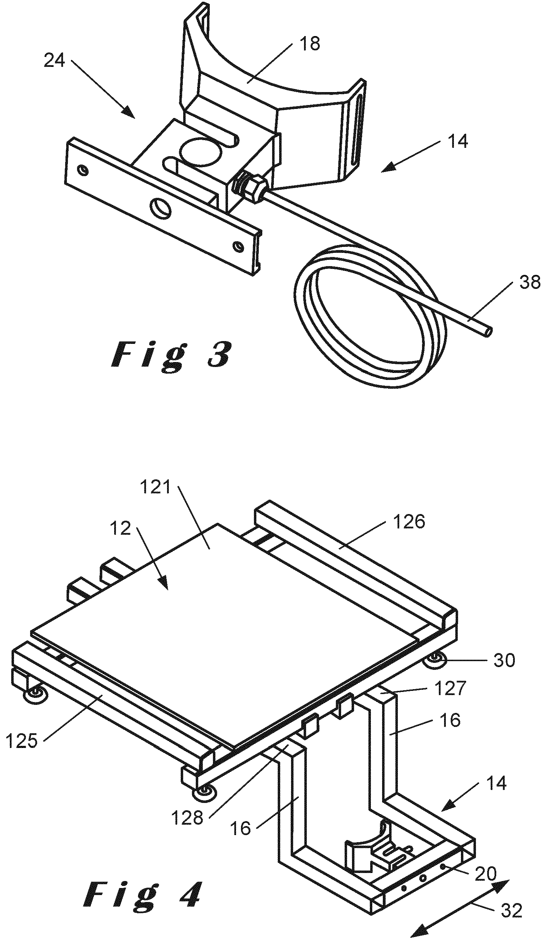

L'instrument est fixé sur une pièce agencée pour être alignée avec la direction de la force exercée par le membre inférieur au niveau de l'élément d'appui du membre inférieur. Cette position de l'instrument de mesure contribue avantageusement à la précision des mesures. En effet, il enregistre de façon particulièrement directe et exacte la force exercée par le membre inférieur au niveau de l'élément d'appui étant donné qu'il est localisé dans la ligne de force, typiquement près de l'élément d'appui. Ce n'est donc pas une force intermédiaire représentative de cette force qui mesurée à une autre position, mais bien la force exacte exercée par le membre inférieur au niveau exact de l'élément d'appui. Il s'agit d'une caractéristique avantageuse vu que l'objet de la présente invention est de fournir un dispositif de mesure d'une force musculaire fournissant des mesures précises. L'instrument de mesure est une même pièce avec l'élément d'appui (visible en figure 3 ci-après commentée).

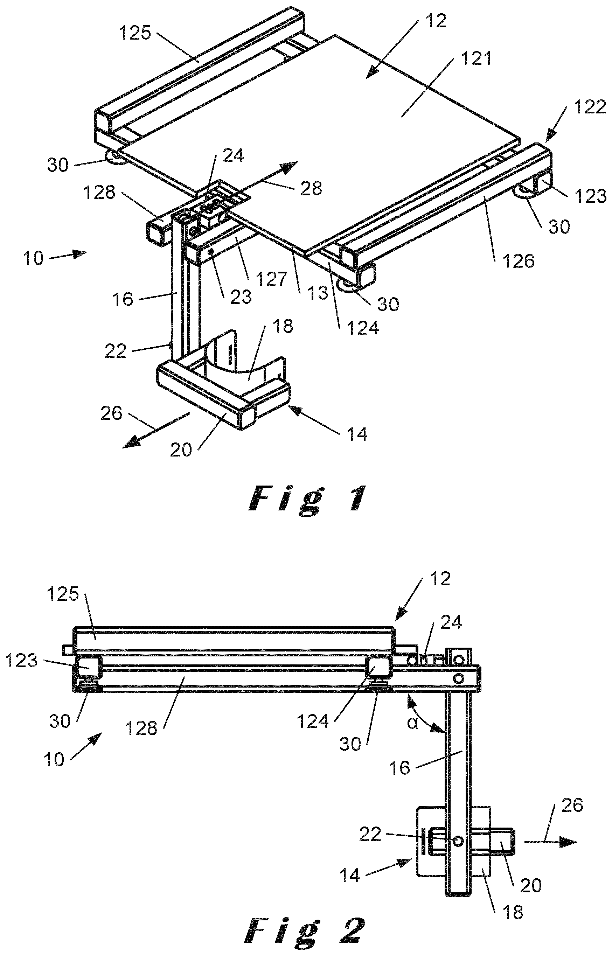

La figure 1 montre une vue en perspective d'un dispositif 10 selon une variante de l'invention du point de vue du positionnement de l'instrument de mesure tel qu'il est expliqué ci-après.

Le dispositif 10 permet parfaitement de mesurer une force musculaire d'un membre inférieur d'un patient. Il peut s'agir par exemple d'un muscle de la cuisse, en particulier le quadriceps ou les ischio-jambiers. Il comporte une assise 12 pour recevoir le patient en position assise, ce qui contribue à obtenir des mesures précises de la force musculaire de muscles du membre inférieur. L'assise 12 est apte à être posée sur un support essentiellement horizontal ; il peut s'agir d'une table ou d'une chaise. L'assise 12 est dépourvue de support au sol, ce qui rend le dispositif simple et léger à transporter. Contrairement aux dispositifs de l'état de la technique, le fait que le dispositif ne comporte pas de pied pour le maintien au sol allège sa structure et le rend portatif. En d'autres termes, l'assise 12 est amovible du support et est apte à être posée sur tout autre support essentiellement horizontal - les mesures n'ont pas à être prises dans un endroit dédié.

L'assise 12 est par exemple un plateau 121 pour recevoir le patient, possiblement rembourrée pour plus de confort du patient. La surface de l'assise 12 est telle que l'entièreté de la cuisse du patient repose sur l'assise et que l'arrière de son genou soit en contact d'un bord de l'assise 12. Le bord est suffisamment arrondi pour épouser l'arrière du genou et assurer un confort au patient. Le plateau 121 mesure environ 40 à 70 cm de côté - de préférence entre 50 et 60 cm de côté pour avoir un équilibre entre encombrement du dispositif 10 et confort du patient en position assise. L'assise 12 peut comporter en outre un châssis 122 auquel est fixé le plateau 121. Le châssis 122 confère la rigidité à l'assise 12. Le châssis 122 est par exemple une ossature formée par deux premières barres 122, 123 supportant le plateau 121 par sa face inférieure et deux autres barres 124, 125 transversalement aux premières barres 122, 123 assurant la rigidité de l'ensemble.

Le dispositif 10 comprend en outre un élément 14 d'appui du membre inférieur pour recevoir au moins une partie du membre inférieur du patient, en particulier la jambe (partie du membre inférieur entre le genou et la cheville), la cheville ou le pied. L'élément 14 est couplé mécaniquement à l'assise 12. L'élément 14 d'appui de la jambe, de la cheville ou du pied, permet le maintien du membre inférieur en place durant la prise de mesure, ce qui permet de fournir des mesures précises. Le membre inférieur est immobilisé dans l'élément 14 pour éviter un déplacement relatif entre l'élément 14 et le membre inférieur comme cela se produit dans les dispositifs de l'art antérieur lorsque le membre inférieur est simplement en appui contre un coussin.

L'élément 14 peut recevoir une partie avant du membre inférieur, sous le genou - par exemple au niveau du tibia. L'élément 14 d'appui du membre inférieur est par exemple une anse 18. L'anse 18 comprend une partie arrondie épousant la courbure de la partie du membre inférieur prise dans l'élément 14 tout en immobilisant latéralement le membre inférieur. L'anse 18 peut être maintenue par une armature 20 permettant de rigidifier la zone de sollicitation du dispositif par le membre inférieur du patient. La figure 3 montre un autre exemple de l'élément 14. Le membre inférieur s'appuie contre la partie arrondie de l'anse 18. Dans ces exemples de réalisation, une sangle peut être prévue pour mieux immobiliser le membre inférieur contre l'élément 14 d'appui. Un fil 38 transfère les signaux de mesure vers une unité de traitement des mesures.

L'élément 14 est couplé mécaniquement à l'assise 12 par exemple par au moins un bras 16. Le ou les bras 16 s'étendent en dehors du plan de l'assise 12. Lorsque l'assise 12 est posée sur un support essentiellement horizontal, le ou les bras 16 déportent l'élément 14 d'appui du membre inférieur sous le plan de l'assise 12. En position assise du patient, l'élément 14 est déporté sous son genou. Selon la longueur du ou des bras 16, l'élément 14 d'appui du membre inférieur peut être au niveau de la cheville, du tibia ou du pied. Selon la figure 1, un seul bras 16 couple mécaniquement l'élément 14 à l'assise 12 ; ceci rend le dispositif plus léger. Selon la figure 4, une paire de bras 16 couplent mécaniquement l'élément 14 à l'assise 12, de part et d'autres de l'élément 14 ; ceci assure plus de stabilité à l'élément 14. Le ou les bras 16 sont orthogonaux au plan de l'assise 12, mais comme cela sera décrit par la suite, l'angle entre le ou les bras 16 et l'assise 12 peut être autre.

Le ou les bras 16 relient mécaniquement l'élément 14 à l'assise 12 par le châssis 122. Le châssis 122 peut comporter des barres 127, 128 auxquelles le ou les bras 16 sont fixés. Le ou les bras 16 sont fixés aux barres 127, 128 selon un axe 23.

Un exemple de couplage mécanique de l'élément 14 d'appui du membre inférieur à l'assise 12 est mieux visible sur la figure 2 qui montre une vue de profil du dispositif 10. Le bras 16 déporte l'élément 14 en dehors du plan de l'assise 12, sous cette dernière lorsque le dispositif 10 est en position d'utilisation sur un support essentiellement horizontal. Les barres 127, 128 sont positionnées sous le plateau 121 et décalent le bras 16 au-delà du plateau 121, vers l'avant de l'assise 12. Ceci permet au patient de s'assoir sur l'assise 12, l'arrière du genou contre un bord avant 13 du plateau 121 et d'avoir l'élément 14 d'appui du membre inférieur en regard d'une partie de son membre inférieur sous le genou. Les barres 127, 128 sont fixées aux barres 123, 124, parallèlement au barres 125, 126. Les barres 127, 128 s'étendent en direction de l'avant du dispositif 10, en saillie d'un bord avant 13 du dispositif. L'élément 14 peut être fixé au bras 16 par un axe 22. L'axe 22 solidarise l'armature 20 au bras 16.

La figure 4 montre une vue en perspective d'un autre exemple de réalisation du dispositif 10. Dans cet exemple, le dispositif comporte deux bras 16 couplant mécaniquement l'élément 14 à l'assise 12 - le reste du dispositif étant le même que sur les figures 1 et 2. Les bras 16 déportent l'élément 14 en dehors du plan de l'assise 12, sous cette dernière lorsque le dispositif 10 est en position d'utilisation sur un support essentiellement horizontal. Chaque bras 16 est relié à l'une des barres 127, 128. Les barres 127, 128 sont positionnées sous le plateau 121 et décalent les bras 16 au-delà du plateau 121, vers l'avant de l'assise 12. Ceci permet au patient de s'assoir sur l'assise 12, l'arrière du genou contre un bord avant 13 du plateau 121 et d'avoir l'élément 14 d'appui du membre inférieur en regard d'une partie de son membre inférieur sous le genou. Les barres 127, 128 sont fixées aux barres 123, 124, parallèlement au barres 125, 126. Les barres 127, 128 s'étendent en direction de l'avant du dispositif 10, en saillie d'un bord avant 13 du dispositif. Dans cet exemple, l'élément 14 est couplé mécaniquement à l'assise par un cadre en 'L' (ou en 'S' ou 'Z' selon la figure 4) formé par les barres 127, 128, les bras 16 et l'armature 20. L'élément 14 peut être fixé aux bras 16 par un axe 22 non visible sur le figure 4. L'axe 22 solidarise l'armature 20 aux bras 16.

L'élément 14 d'appui peut être couplé par le bras 16 unique ou par deux bras 16 formant le cadre décrit sur la figure 4, mais positionné(s) sous l'assise 12 et non au-delà de celle-ci comme visible sur les figures. Le dispositif 10 est alors placé sur un support disposant d'un dégagement par dessous. Le patient exerce alors une force selon un sens opposé à celui de la flèche 26 des figures 1 et 2.

De préférence, le dispositif ne comprend qu'un seul élément 14 d'appui du membre inférieur, ajustable sur le bras 16 pour s'adapter à différents membres inférieurs. De sorte à encore simplifier la structure du dispositif, et faciliter son transport, l'élément 14 peut être utilisé pour un membre inférieur ou l'autre. L'élément 14 peut être démonté et placé d'un côté ou de l'autre du bras 16 des figures 1 et 2, en regard du membre inférieur dont on souhaite mesurer la force musculaire. L'axe 22 est par exemple une vis maintenant de manière amovible l'élément 14 en place sur le bras. Il est aussi envisageable d'ajuster la position de l'élément 14 en hauteur le long du bras 16 pour s'adapter à la taille des membres inférieurs du patient.

Le dispositif 10 comporte en outre un instrument 24 de mesure pour mesurer une force exercée par le membre inférieur au niveau de l'élément 14 d'appui du membre inférieur. L'instrument de mesure 24 peut être une jauge de contrainte ou un dynamomètre mécanique. L'instrument de mesure 24 peut travailler en traction ou en compression. Selon diverses variantes de l'invention, l'instrument de mesure 24 peut être positionné en de nombreux endroits du dispositif 10, tant que l'effort du membre inférieur au niveau de l'élément 14 d'appui du membre inférieur est transféré à l'instrument 24 de mesure. Par exemple, l'instrument de mesure 24 pourrait être situé dans le ou les bras 16 pour mesurer la déformation des bras 16. Comme cela est visible sur les figures 1 et 2, l'instrument de mesure 24 pourrait aussi être au niveau de l'assise 12, entre les barres 127, 128, en coopération avec le bras 16: la sollicitation de l'élément 14 d'appui par le membre inférieur se propagerait alors dans le bras 16 qui à son tour solliciterait l'instrument 24 de mesure.

Selon la figure 3, dans une réalisation de l'invention, l'instrument de mesure 24 et l'élément d'appui 14 sont une seule et même pièce. Dans cette configuration, l'instrument de mesure 24 enregistre exactement la force exercée par le membre inférieur puisqu'il est localisé au plus près de celui-ci. Une structure en forme de S se déforme par sollicitation du membre inférieur. L'instrument de mesure 24 travaille en compression.

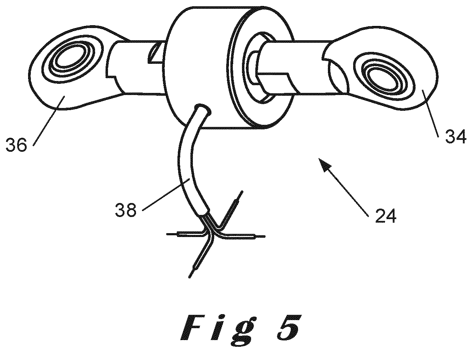

La figure 5 montre une vue en perspective d'un autre exemple d'instrument de mesure 24. Dans cette réalisation non couvert par la présente invention, l'instrument de mesure 24 est placé derrière le membre inférieur, typiquement dans la ligne de force. L'instrument de mesure 24 fonctionne dès lors en traction. Il est articulé sur deux rotules 34, 36 afin d'accompagner le mouvement du membre inférieur quelle que soit la direction de ce dernier. Sur les figures 1, 2, 4, l'élément 14 en appui sur l'avant du membre inférieur, est relié à la rotule 34 ou 36 de l'instrument 24 de mesure situé derrière le membre inférieur, l'autre rotule 34, 36 étant reliée à une armature additionnelle du type l'armature 20. Un fil 38 transfère les signaux de mesure vers une unité de traitement des mesures.

L'instrument 24 de mesure peut être couplé au bras 16 unique des figures 1 et 2 ou aux deux bras 16 de la figure 4, en étant positionné sous l'assise 12 et non au-delà de celle-ci. L'assise 12 est alors placée sur un support disposant d'un dégagement par dessous.

Dans une configuration normale d'utilisation, le dispositif 10 est configuré de sorte que la force exercée par le membre inférieur au niveau de l'élément 14 d'appui du membre inférieur est essentiellement parallèle à l'assise 12. Sur les figures 1 et 2, la flèche 26 montre le sens d'application d'une force par le membre inférieur du patient au niveau de l'élément 14 d'appui du membre inférieur. Grâce au poids du patient s'exerçant au niveau de l'assise 12, le dispositif 10 de mesure reste essentiellement immobile par rapport au support sur lequel est posé l'assise, lors de l'application de cette force. Dans la configuration des figures 1 et 2, sur lesquelles le bras 16 s'étend orthogonalement à l'assise 12, la flèche 26 est dans un plan orthogonal à l'assise 12 comprenant le bras 16 et, dans ce même plan, une flèche 28 montre la force qui s'applique en réaction sur l'instrument 24 positionné sous l'assise 12.

Selon l'invention, l'instrument 24 est fixé sur une pièce alignée avec la direction de la force exercée par le membre inférieur, typiquement suite à une contraction de la cuisse. L'immobilité du dispositif assuré par le poids du patient permet non seulement la facilité de la prise de mesures mais aussi la précision des mesures.

L'élément 14 d'appui et l'instrument 24 de mesure peuvent être placés chacun par rapport au membre inférieur selon plusieurs combinaisons. L'élément 14 peut être en appui sur le membre inférieur par devant ou derrière le membre ; l'instrument 24 de mesure peut être aussi devant ou derrière le membre et travailler en traction ou compression. Le dispositif offre donc une variété de structures permettant de s'adapter à différents besoins de mesure. De préférence, le long de la ligne de force, les éléments suivants sont agencés et en contact entre eux dans un des ordres suivants, selon le sens de la force :
- instrument de mesure, (partie du) membre inférieur, élément d'appui ;
- (partie du) membre inférieur, élément d'appui, instrument de mesure.

Le dispositif 10 peut comprendre des organes 30 pour le positionnement amovible de l'assise sur le support. Il peut s'agir d'organes 30 réglables en hauteur afin d'assurer la stabilité du dispositif sur le support. En outre, les organes 30 peuvent être des ventouses ou des étaux afin de maintenir le dispositif en une même position sur le support.

Le ou les bras 16 peuvent être articulés par rapport à l'assise 12, par exemple autour de l'axe 23. Comme cela est visible sur la figure 2 (mais applicable à la figure 4 aussi), l'angle α entre le bras 16 et l'assise 12 peut être réglable, dans une configuration d'utilisation du dispositif. Une fois que l'angle α souhaité est obtenu, le bras 16 est immobilisé par rapport à l'assise 12 pour la prise de mesure. Les figures 1 et 2 montrent un angle α de 90° ; il peut varier entre 30° (position dans laquelle le bras 16 s'étend sous l'assise) et 180° (position dans laquelle le bras 16 s'étend dans le plan de l'assise 12, devant elle). La variation de l'angle α est utile pour prendre des mesures dans divers positions du membre inférieur. Le bras 16 peut aussi être plié contre l'assise 12 dans une configuration de transport du dispositif 10 - ce qui facilite encore le transport du dispositif 10. L'angle α est alors proche de 0°.

Sur l'exemple de réalisation de la figure 4, le patient se place à gauche ou à droite de l'assise 12 pour tester le membre inférieur droit ou gauche. Sur la figure 4, les bras 16 peuvent aussi être mobiles par rapport à l'assise 12, en particulier en translation latéralement par rapport au patient, selon la double flèche 32. Les bras 16 peuvent être positionnés plus à gauche ou plus à droite de l'assise 12 selon que l'on veuille tester le membre inférieur gauche ou droit. Sur les figures 1 et 2, il est aussi envisageable que le patient se place à gauche ou à droite de l'assise 12 pour tester le membre inférieur droit ou gauche ; la mobilité en translation latérale du bras 16 selon la double flèche 32 est aussi applicable sur l'exemple des figures 1 et 2.

Le fonctionnement du dispositif va maintenant être décrit en lien avec la description d'un procédé de mesure d'une force musculaire d'un membre inférieur d'un patient. Le procédé comprend une étape de fourniture du dispositif 10 tel que décrit précédemment. L'assise 12 est mise en place sur un support essentiellement horizontal ; il peut s'agir d'une table ou d'une chaise. Le patient s'assoit ensuite sur l'assise 12, regardant vers l'avant du dispositif, puis son membre inférieur dont la force est à mesurer est positionné dans l'élément 14 d'appui du membre inférieur. Les mesures de force sont ensuite prises par l'instrument 24 de mesure au niveau de l'élément 14. Pour cela, le patient contracte et relâche sa cuisse, de manière répétée ; cela tend à provoquer un mouvement du bas du membre inférieur, sous le genou, vers l'avant du patient, ce qui sollicite l'élément 14. L'instrument 24 prend les mesures qui sont envoyées vers une unité de traitement des mesures. Le procédé est simple car le dispositif 10 peut être transporté en un lieu quelconque et placé sur tout support essentiellement horizontal ; les mesures sont précises car le dispositif de mesure reste essentiellement immobile par rapport au support lors de l'application de la force par le membre inférieur au niveau de l'élément d'appui du membre inférieur, grâce au poids du patient s'exerçant au niveau de l'assise 12.

Le positionnement du patient sur l'assise 12 est tel que son pied soit à distance du sol. Le support est donc choisi de sorte que la hauteur entre la surface de l'assise 12 posée sur ce support et le sol soit supérieure à la longueur du membre inférieur du patient sous son genou. Ceci permet d'éviter de fausser les mesures par frottement du pied sur le sol. Selon la figure 2, le patient est positionné de sorte que le bras 16 soit entre ses membres inférieurs ; selon la figure 4, l'un des bras 16 est entre ses membres inférieurs. L'entièreté de sa cuisse repose sur l'assise 12 et l'arrière de son genou est en contact du bord avant 13 de l'assise 12. Le bord avant 13 de l'assise 12 est un point d'appui pour l'exercice de la force par le membre inférieur suite à la contraction de la cuisse ; la contraction de la cuisse provoque la rotation du bas du membre inférieur vers l'avant du dispositif et du patient, autour du genou en appui sur le bord avant 13. Il n'est donc pas nécessaire d'avoir un point d'appui spécifique supplémentaire pour les prises de mesure. Une telle position permet la reproductibilité des mesures car il n'y a pas de réglage à faire sur l'assise 12 ou sur la prise d'appui. En outre, pour assurer la précision des mesures, le patient se tient assis dans une position avec le dos droit. Ainsi, la position assise du patient garantit l'angulation. La cuisse posée sur l'assise 12 est parallèle à l'horizontale et le tronc à la verticale ; l'angle de la cuisse et le bas du membre inférieur est garanti.

Le procédé peut comprendre une étape de positionnement de l'élément 14 d'appui du membre inférieur en regard du membre inférieur dont la force musculaire est à mesurer. Dans la configuration des figures 1 et 2, le même élément 14 peut être utilisé pour les deux membres inférieurs du patient ; l'élément 14 est donc monté du côté du bras 16 en regard du membre inférieur à tester et la hauteur de l'élément 14 le long du bras 16 est ajustée à la taille du membre inférieur du patient. Sur la figure 4, c'est le patient qui se place sur l'assise 12 de manière à positionner le membre inférieur à tester en face de l'élément d'appui 14. En outre, sur les figures 1, 2, 4, l'élément 14 d'appui peut être positionné en regard du membre inférieur dont la force musculaire est à mesurer par translation latérale du ou des bras.

Selon le type de mesures à effectuer, il est possible de régler l'angle α entre l'assise 12 et le ou les bras 16 couplant mécaniquement l'élément 14 d'appui du membre inférieur à l'assise 12. Egalement, comme décrit précédemment, l'élément 14 d'appui peut être devant ou derrière le membre inférieur, le patient sollicitant l'élément vers l'avant ou l'arrière. Ceci permet de prendre d'autres types de mesures.

Le procédé permet de mesurer la fatigue d'un muscle d'un membre inférieur, tel que le quadriceps ou les ischio-jambiers . Le dispositif assure la prise de mesure de l'ensemble du quadriceps ou des ischio-jambiers.

Le procédé peut aussi comprendre une étape d'électrostimulation engendrant une force exercée par le membre inférieur au niveau de l'élément 14 d'appui du membre inférieur, comme cela est décrit ci-dessus.

Plus précisément, le procédé comprend de préférence les étapes suivantes :
- électrostimulations d'un muscle du membre inférieur à différentes fréquences,
- prise de mesures de forces du membre inférieur au niveau de l'élément 14 d'appui du membre inférieur en réponse aux électrostimulations susdites,
- détermination d'une fatigue musculaire sur bases des mesures de forces du membre inférieur prises en réponse aux électrostimulations susdites.

Ainsi, grâce au dispositif 10 et au procédé selon ce mode de réalisation préféré, il est possible de déterminer la fatigue musculaire du muscle de façon simple, sûre et fiable. En effet, l'utilisation d'électrostimulations permet de stimuler le muscle quelle que soit sa fatigue et de faire développer au muscle une force involontaire en réponse aux électrostimulations. Cette étape peut alors être effectuée à tout moment, également après un entraînement sportif, sans mettre le sujet en danger de blessure, indépendamment de la volonté du sujet. Pour un nombre réduit et une fréquence adaptée d'électrostimulations, ce procédé n'induit pas de fatigue musculaire additionnelle, et ne fausse donc pas la détermination de la fatigue musculaire éventuelle préexistante. En particulier, la fatigue musculaire avant et après une exécution du procédé de détermination est sensiblement la même. Ce procédé permet de déterminer efficacement la fatigue musculaire car elle déforme non uniformément la courbe de la force développée par le muscle, et donc le membre inférieur, en réponse à une électrostimulation à une fréquence en fonction de cette fréquence. Il est donc possible de déterminer la fatigue musculaire sur base des mesures de forces prises pour différentes fréquences, par exemple en comparant ces mesures de forces. Ceci présente l'avantage d'être indépendant du contexte d'exécution de ce procédé. En particulier, aucune comparaison avec une telle courbe standard connue au repos pour le patient, aucune mesure préalable et aucune condition d'exécution spécifique ne sont nécessaires. De préférence, les fréquences sont entre 0 et 1 kHz, préférentiellement plus petites que 500 Hz, plus préférentiellement plus petite que 200 Hz. Les fréquences comprennent de préférence une première et une deuxième fréquences, µ et µ', différant d'au moins 10% l'une de l'autre. Dans ce cas, les mesures de forces comprennent une première mesure de force F1 du membre inférieur au niveau de l'élément 14 d'appui du membre inférieur en réponse à l'électrostimulation du muscle à la première fréquence, et une deuxième mesure de force F2 du membre inférieur au niveau de l'élément 14 d'appui du membre inférieur en réponse à l'électrostimulation du muscle à la deuxième fréquence. La détermination de la fatigue musculaire comprend alors de préférence un calcul d'un ratio F1/F2 et est basée au moins sur une comparaison entre ce ratio et un seuil. Ce seuil peut être de la forme F(µ)/F(µ') où F est une fonction indépendante du patient exprimant la force développée par un muscle non fatigué en réponse à une électrostimulation de ce muscle à une fréquence, en fonction de celle-ci. Par exemple, µ est entre 0 et 50 Hz, préférentiellement, entre 10 et 40 Hz, plus préférentiellement d'environ 20Hz ; µ' est comprise entre 50 et 150 Hz, préférentiellement entre 90 Hz et 120 Hz, plus préférentiellement d'environ 100 Hz ; et le seuil est compris entre 50 et 100%, préférentiellement entre 70 et 90%, plus préférentiellement, il est d'environ 80% lorsque µ est d'environ 20 Hz et µ' est d'environ 100 Hz. Cette réalisation a l'avantage d'être simple et de permettre un calcul rapide et peu complexe pour déterminer la fatigue musculaire. Elle est aussi très efficace. En effet, comme µ diffère d'au moins 10% de la deuxième fréquence, le rapport est pleinement affecté par la non uniformité et non linéarité de la déformation de la courbe en fonction de la fatigue musculaire. Notamment, F2 correspond assez approximativement à F(µ') par exemple pour µ' = 100 Hz (ou plus grand), alors que F1 est d'autant plus éloignée de F(µ) qu'elle est affectée par la fatigue du muscle, pour une fréquence µ suffisamment plus petite que µ', par exemple entre 10 et 30 Hz. Dès lors, lorsque la comparaison du ratio F1/F2 avec le seuil permet d'identifier une différence, celle-ci exprime une fatigue musculaire qui peut être déterminée implicitement et/ou explicitement.

De préférence, µ est inférieure à 50 Hz, et les fréquences comprennent une famille de fréquences inférieures à 200 Hz étant multiples entiers de la première fréquence, et préférentiellement toutes telles fréquences. La détermination de la fatigue musculaire peut alors se baser sur un calcul d'une intégrale discrète d'une fonction associant à chaque fréquence de la famille une mesure de force prise au niveau de l'élément 14 d'appui en réponse à l'électrostimulation à cette fréquence. Cette intégrale discrète correspond typiquement à une somme de Riemann. De préférence, µ est inférieure à 20 Hz, de préférence inférieure à 10 Hz, pour une bonne précision de calcul. De préférence, une comparaison de l'intégrale discrète calculée est effectuée avec une valeur d'aire attendue, et la détermination de la fatigue musculaire est basée sur cette comparaison. La valeur d'aire peut être l'aire sous le graphe de la fonction F susdite. Comme bien connu en calcul discret, la comparaison permet alors d'évaluer une différence entre cette aire théorique pour un muscle non fatigué et ses approximations par une somme de Riemann pour le muscle considéré, et de déterminer la fatigue musculaire sur cette base de manière précise en raison du nombre et de la répartition uniforme globale des fréquences de la famille.

De façon générale, la fatigue musculaire peut être déterminée par calcul(s) sur les mesures de forces prises (par exemple, par ratio de deux forces, par calcul intégral discret, comme susmentionnés, et/ou par combinaison de ces techniques) et/ou comparaison d'au moins un tel calcul à au moins une valeur attendue.

La présente invention a été décrite en relation avec des modes de réalisations spécifiques, qui ont une valeur purement illustrative et ne doivent pas être considérés comme limitatifs.

D'une manière générale, il apparaîtra évident pour un homme du métier que la présente invention n'est pas limitée aux exemples illustrés et/ou décrits ci-dessus.

## Revendications

1. Dispositif (10) de mesure d'une force musculaire d'un membre inférieur d'un patient comprenant :
• une assise (12) pour recevoir le patient en position assise et apte à être posée de façon amovible sur un support essentiellement horizontal,
• un élément (14) d'appui du membre inférieur pour recevoir au moins une partie du membre inférieur du patient, couplé mécaniquement à l'assise,
• un instrument (24) de mesure pour mesurer une force exercée par le membre inférieur au niveau de l'élément d'appui du membre inférieur, l'instrument (24) étant fixé sur une pièce agencée pour être alignée avec la direction de cette force, le dispositif (10) de mesure étant configuré de sorte qu'il reste essentiellement immobile par rapport au support lors de l'application d'une force par le membre inférieur du patient au niveau de l'élément (14) d'appui du membre inférieur, grâce au poids du patient s'exerçant au niveau de l'assise (12),
**caractérisé en ce que** l'instrument (24) de mesure est une même pièce avec l'élément (14) d'appui.

2. Dispositif (10) selon la revendication 1, dans lequel l'assise (12) est un plateau (121), de préférence fixé à un châssis (122), et s'étendant continument d'au moins 40 centimètres selon deux axes perpendiculaires.

3. Dispositif (10) selon la revendication 1 ou 2, dans lequel l'assise (12) est dépourvue de support au sol.

4. Dispositif (10) selon l'une des revendications précédentes, comprenant au moins un bras (16) couplant mécaniquement l'élément (14) d'appui du membre inférieur à l'assise (12), et déportant l'élément (14) en dehors du plan de l'assise (12), sous l'assise, en configuration d'utilisation du dispositif.

5. Dispositif (10) selon la revendication 4, dans lequel le ou les bras (16) sont mobiles en translation latéralement par rapport à l'assise (12).

6. Dispositif (10) selon l'une des revendications précédentes, dans lequel l'instrument (24) de mesure est une jauge de contrainte ou un dynamomètre mécanique.

7. Dispositif (10) selon l'une des revendications précédentes, comprenant en outre des organes (30) consistant en des ventouses ou des étaux pour un positionnement amovible de l'assise (12) sur le support.

8. Dispositif (10) selon l'une des revendications précédentes, configuré de sorte que l'élément (14) d'appui du membre inférieur est apte à recevoir une partie du membre inférieur sous le genou du patient, et dans lequel l'élément (14) d'appui est une anse (18) conformée pour s'adapter à la partie du membre inférieur en appui contre l'élément (14).

9. Procédé de mesure d'une force musculaire d'un membre inférieur d'un patient, comprenant les étapes suivantes :
• fourniture du dispositif (10) selon l'une des revendications précédentes,
• mise en place de l'assise (12) sur un support essentiellement horizontal,
• positionnement du patient assis sur l'assise (12),
• positionnement d'un membre inférieur du patient dont la force est à mesurer dans l'élément (14) d'appui du membre inférieur,
• prise de mesures de force du membre inférieur au niveau de l'élément (14) d'appui du membre inférieur.

10. Procédé selon la revendication 9, dans lequel le support est une table ou une chaise.

11. Procédé selon la revendication 9 ou 10, comprenant en outre une étape d'électrostimulation engendrant une force exercée par le membre inférieur au niveau de l'élément (14) d'appui du membre inférieur.

12. Procédé selon l'une quelconque des revendications 9 à 11, comprenant en outre les étapes suivantes :
• électrostimulations d'un muscle du membre inférieur, par exemple un quadriceps ou un ischio-jambier, à différentes fréquences,
• prise de mesures de forces du membre inférieur au niveau de l'élément (14) d'appui du membre inférieur en réponse aux électrostimulations susdites,
• détermination d'une fatigue musculaire sur bases des mesures de forces du membre inférieur prises en réponse aux électrostimulations susdites.

13. Procédé selon la revendication 12, dans lequel les différentes fréquences comprennent une première et une deuxième fréquences, différant d'au moins 10% l'une de l'autre, et les mesures de forces comprennent une première mesure de force du membre inférieur au niveau de l'élément (14) d'appui du membre inférieur en réponse à l'électrostimulation du muscle à la première fréquence, et une deuxième mesure de force du membre inférieur au niveau de l'élément (14) d'appui du membre inférieur en réponse à l'électrostimulation du muscle à la deuxième fréquence.

14. Procédé selon la revendication 13, dans lequel la détermination de la fatigue musculaire comprend un calcul de ratio entre la première et la deuxième mesures de forces et est basée au moins sur une comparaison entre ce ratio et un seuil.

15. Procédé selon la revendication 13 ou 14, dans lequel la première fréquence est inférieure à 50 Hz, dans lequel les différentes fréquences comprennent une famille de fréquences inférieures à 200 Hz et multiples entiers de la première fréquence, et dans lequel la détermination de la fatigue musculaire est basée sur un calcul d'une intégrale discrète d'une fonction associant à chaque fréquence de la famille une mesure de force prise au niveau de l'élément (14) d'appui du membre inférieur en réponse à l'électrostimulation à cette fréquence.

## Patentansprüche

1. Vorrichtung (10) zum Messen einer Muskelkraft einer unteren Extremität eines Patienten, umfassend:
- eine Sitzfläche (12) zum Aufnehmen des Patienten in sitzender Position, die abnehmbar auf eine im Wesentlichen horizontale Abstützung gesetzt werden kann;
- ein Auflageelement (14) für die untere Extremität zum Aufnehmen mindestens eines Teils der unteren Extremität des Patienten, das mechanisch mit der Sitzfläche gekoppelt ist,
- ein Messinstrument (24) zum Messen einer Kraft, die durch die untere Extremität im Bereich des Auflageelements für die untere Extremität ausgeübt wird, wobei das Instrument (24) an einem Stück befestigt ist, das so eingerichtet ist, dass es mit der Richtung dieser Kraft fluchtet,
wobei die Messvorrichtung (10) so konfiguriert ist, dass sie beim Anlegen einer Kraft durch die untere Extremität des Patienten im Bereich des Auflageelements (14) für die untere Extremität dank des Gewichts des Patienten, das auf den Bereich der Sitzfläche (12) ausgeübt wird, in Bezug auf die Abstützung im Wesentlichen unbeweglich bleibt,
**dadurch gekennzeichnet, dass** das Messinstrument (24) mit dem Auflageelement (14) einstückig ist.

2. Vorrichtung (10) nach Anspruch 1, wobei die Sitzfläche (12) eine Platte (121) ist, die bevorzugt an einem Rahmen (122) befestigt ist und sich durchgehend um mindestens 40 Zentimeter entlang von zwei senkrechten Achsen erstreckt.

3. Vorrichtung (10) nach Anspruch 1 oder 2, wobei die Sitzfläche (12) keine Abstützung auf dem Boden aufweist.

4. Vorrichtung (10) nach einem der vorstehenden Ansprüche, die mindestens einen Arm (16), der das Auflageelement (14) für die untere Extremität mechanisch mit der Sitzfläche (12) koppelt und das Element (14) in der Gebrauchskonfiguration der Vorrichtung unterhalb der Sitzfläche aus der Ebene der Sitzfläche (12) verlagert.

5. Vorrichtung (10) nach Anspruch 4, wobei der oder die Arme (16) in Bezug auf die Sitzfläche (12) seitlich verschiebebeweglich ist (sind).

6. Vorrichtung (10) nach einem der vorstehenden Ansprüche, wobei das Messinstrument (24) ein Dehnungsmessstreifen oder ein mechanisches Dynamometer ist.

7. Vorrichtung (10) nach einem der vorstehenden Ansprüche, die weiter Organe (30) umfasst, die aus Saugnäpfen oder Klemmen für eine abnehmbare Positionierung der Sitzfläche (12) auf der Abstützung bestehen.

8. Vorrichtung (10) nach einem der vorstehenden Ansprüche, die so konfiguriert ist, dass das Auflageelement (14) für die untere Extremität einen Teil der unteren Extremität unterhalb des Knies des Patienten aufnehmen kann, und wobei das Auflageelement (14) ein Bügel (18) ist, der so ausgestaltet ist, dass er sich an den Teil der unteren Extremität, der an dem Element (14) aufliegt, anpasst.

9. Verfahren zum Messen einer Muskelkraft einer unteren Extremität eines Patienten, das die folgenden Schritte umfasst:
- Bereitstellen der Vorrichtung (10) nach einem der vorstehenden Ansprüche,
- Platzieren der Sitzfläche (12) auf einer im Wesentlichen horizontalen Abstützung,
- Positionieren des Patienten sitzend auf der Sitzfläche (12),
- Positionieren einer unteren Extremität des Patienten, deren Kraft gemessen werden soll, in dem Auflageelement (14) für die untere Extremität,
- Vornehmen von Kraftmessungen der unteren Extremität im Bereich des Auflageelements (14) für die untere Extremität.

10. Verfahren nach Anspruch 9, wobei die Abstützung ein Tisch oder ein Stuhl ist.

11. Verfahren nach Anspruch 9 oder 10, das weiter einen Schritt der Elektrostimulation umfasst, die eine Kraft erzeugt, die durch die untere Extremität im Bereich des Auflageelements (14) für die untere Extremität ausgeübt wird.

12. Verfahren nach einem der Ansprüche 9 bis 11, das weiter die folgenden Schritte umfasst:
- Elektrostimulationen eines Muskels der unteren Extremität, zum Beispiel eines Quadrizeps oder eines Oberschenkelmuskels, mit unterschiedlichen Frequenzen,
- Vornehmen von Kraftmessungen der unteren Extremität im Bereich des Auflageelements (14) für die untere Extremität in Reaktion auf die vorgenannten Elektrostimulationen,
- Bestimmen einer Muskelermüdung auf Basis der Kraftmessungen der unteren Extremität, die in Reaktion auf die vorgenannten Elektrostimulationen vorgenommen wurden.

13. Verfahren nach Anspruch 12, wobei die unterschiedlichen Frequenzen eine erste und eine zweite Frequenz umfassen, die sich um mindestens 10 % voneinander unterscheiden, und die Kraftmessungen eine erste Kraftmessung der unteren Extremität im Bereich des Auflageelements (14) für die untere Extremität in Reaktion auf die Elektrostimulation des Muskels mit der ersten Frequenz, und eine zweite Kraftmessung der unteren Extremität im Bereich des Auflageelements (14) für die untere Extremität in Reaktion auf die Elektrostimulation des Muskels mit der zweiten Frequenz umfassen.

14. Verfahren nach Anspruch 13, wobei das Bestimmen der Muskelermüdung eine Berechnung des Verhältnisses zwischen der ersten und der zweiten Kraftmessung umfasst und mindestens auf einem Vergleich zwischen diesem Verhältnis und einer Schwelle basiert.

15. Verfahren nach Anspruch 13 oder 14, wobei die erste Frequenz kleiner als 50 Hz ist, wobei die unterschiedlichen Frequenzen eine Gruppe von Frequenzen kleiner als 200 Hz und ganzzahlige Vielfache der ersten Frequenz umfassen, und wobei das Bestimmen der Muskelermüdung auf einer Berechnung eines diskreten Integrals einer Funktion basiert, die jeder Frequenz der Gruppe eine Kraftmessung zuordnet, die im Bereich des Auflageelements (14) für die untere Extremität in Reaktion auf die Elektrostimulation mit dieser Frequenz vorgenommen wird.

## Claims

1. A device (10) for measuring a muscular force of a lower limb of a patient comprising:
- a sitting (12) for receiving the patient in a seated position and adapted to be removably placed on a substantially horizontal support,
- a support element (14) for supporting the lower limb in order to receive at least a portion of the lower limb of the patient, mechanically coupled to the sitting,
- a measurement instrument (24) for measuring a force exerted by the lower limb at the level of the support element for supporting the lower limb,
the instrument (24) being attached to a part arranged to be aligned with the direction of that force,
the measurement device (10) being configured such that it remains substantially immobile relative to the support when a force is applied by the lower limb of the patient at the level of the support element (14) for supporting the lower limb, due to the weight of the patient being exerted at the level of the sitting (12),
**characterized in that** the measurement instrument (24) is a single part with the support element (14).

2. The device (10) according to claim 1, wherein the sitting (12) is a tray (121), preferably attached to a chassis (122), and extending continuously by at least 40 centimetres along two perpendicular axes.

3. The device (10) according to claim 1 or 2, wherein the sitting (12) has no floor support.

4. The device (10) according to any of the preceding claims, comprising at least one arm (16) mechanically coupling the support element (14) for supporting the lower limb to the sitting (12), and offsetting the support element (14) out of the plane of the sitting (12), under the sitting, in a use configuration of the device.

5. The device (10) according to claim 4, wherein the arm or the arms (16) are movable in translation laterally with respect to the sitting (12).

6. The device (10) according to any of the preceding claims, wherein the measurement instrument (24) is a strain gauge or a mechanical dynamometer.

7. The device (10) according to any of the preceding claims, further comprising members (30) consisting of suction cups or clamps for a removable positioning of the sitting (12) on the support.

8. The device (10) according to any of the preceding claims, configured such that the support element (14) for supporting the lower limb is adapted to receive a portion of the lower limb below the knee of the patient, and wherein the support element (14) is a bight (18) conformed to adapt to the portion of the lower limb resting against the support element (14).

9. A method for measuring a muscular force of a lower limb of a patient, comprising the following steps:
- providing the device (10) according to one of the preceding claims,
- placing the sitting (12) on a substantially horizontal support,
- positioning the patient seated on the sitting (12),
- positioning a lower limb of the patient whose force is to be measured in the support element (14) for supporting the lower limb,
- taking force measurements of the lower limb at the level of the support element (14) for supporting the lower limb.

10. The method according to claim 9, wherein the support is a table or a chair.

11. The method according to claim 9 or 10, further comprising an electro-stimulation step generating a force exerted by the lower limb at the level of the support element (14) for supporting the lower limb.

12. The method according to any one of claims 9 to 11, further comprising the following steps:
- electro-stimulating a muscle of the lower limb, for example a quadriceps or a hamstring, at different frequencies,
- taking force measurements of the lower limb at the level of the support element (14) for supporting the lower limb in response to the above-mentioned electro-stimulations,
- determining a muscular fatigue on the basis of the force measurements of the lower limb taken in response to the above-mentioned electro-stimulations.

13. The method according to claim 12, wherein the different frequencies comprise a first frequency and a second frequency, differing by at least 10% from each other, and the force measurements comprise a first force measurement of the lower limb at the level of the support element (14) for supporting the lower limb in response to the electro-stimulation of the muscle at the first frequency, and a second force measurement of the lower limb at the level of the support element (14) for supporting the lower limb in response to the electro-stimulation of the muscle at the second frequency.

14. The method of claim 13, wherein the determination of the muscular fatigue comprises a calculation of ratio between the first and the second force measurements and is based at least on a comparison of that ratio with a threshold.

15. The method according to claim 13 or 14, wherein the first frequency is lower than 50 Hz, wherein the different frequencies comprise a family of frequencies lower than 200 Hz and integer multiples of the first frequency, and wherein the determination of the muscular fatigue is based on a calculation of a discrete integral of a function associating with each frequency of the family a force measurement taken at the level of the support element (14) for supporting the lower limb in response to the electro-stimulation at that frequency.
